# EUROPEAN PATENT SPECIFICATION

(11) **EP 2 455 159 B1**
(45) Date of publication and mention of the grant of the patent: **12.11.2014**
(21) Application number: 10799799.1
(22) Date of filing: 06.07.2010
(51) Int. Cl.: B01J 20/24, A61F 13/15, A61F 13/49, A61F 13/53, C08L 5/00

(54) **ABSORBENT BODY AND ABSORBENT ARTICLE**
SAUGFÄHIGER KÖRPER UND SAUGFÄHIGER ARTIKEL
CORPS ABSORBANT ET ARTICLE ABSORBANT

(30) Priority: 13.07.2009 JP 2009164996
(43) Date of publication of application: 23.05.2012
(73) Proprietor: Unicharm Corporation, Ehime 799-0111 (JP)
(72) Inventor: NAKASHITA, Masashi, Kanonji-shi Kagawa 769-1602 (JP); KONISHI, Takayoshi, Kanonji-shi Kagawa 769-1602 (JP); MIZUTANI, Satoshi, Kanonji-shi Kagawa 769-1602 (JP)
(74) Representative: Knights, Rupert
(86) International application number: PCT/JP2010/061737
(87) International publication number: WO 2011/007741

(56) References cited:
- EP-A1- 1 129 730
- JP-A- H08 510 487
- JP-A- 2003 079 325
- JP-A- 2003 159 528
- JP-A- 2005 186 015
- JP-A- 2005 263 858
- JP-T- 2003 533 285

## Description

### [Technical Field]

The present invention relates to an absorbent core capable of promoting and/or inhibiting gelling, as well as to an absorbent article containing the absorbent core.

### [Background Art]

Absorbent cores, and absorbent articles containing them, are used in disposable diapers, sanitary products, medical blood absorbent articles, pet rearing products and the like for treatment of body fluids and excreted fluids, and they are required to have excellent water absorption performance. Absorbent cores, and absorbent articles containing them, must also be lightweight and thin, and from the viewpoint of environmental considerations and hygiene, their water disintegratability and biodegradability have been studied to reduce incineration after use and suitability for disposal in water toilets.

Acrylic acid-based absorbers are known as absorbers that can absorb water at several hundred to several thousand times their dry mass, and they are used in a number of products. In particular, absorbent cores wherein super-absorbent polymer particles composed of a salt of crosslinked polyacrylic acid are dispersed in pulp fiber are widely used in the fields of disposable diapers, sanitary products, medical blood absorbent articles and pet rearing products.

However, with acrylic acid-based absorbers, the liquid, such as body fluid or excreted fluid is absorbed, held and/or immobilized after reaching the location of the absorber, and time is required until all of the liquid reaches the location of the absorber, such that liquid leakage can occur before immobilization of the liquid. In addition, a relatively large amount of the acrylic acid-based absorber must be used to obtain satisfactory absorption performance, which leads to difficulty in achieving "lighter mass of the absorbent core and absorbent article".

Another property of the acrylic acid-based absorber is that its volume increases as the absorber particles incorporate air from the surroundings after liquid absorption, and this tends to increase the thickness after absorption, often leading to "wear discomfort" and/or "reduced absorption performance". Although an acrylic acid-based absorber has a very high absorption rate for ion-exchanged water, its absorption rate for ion-containing liquids, such as body fluids tends to be much lower. Attempts have been made to lower the crosslinking degree of the acrylic acid-based absorber to increase absorption performance, but this leads to problems, such as reduced gel strength. From the viewpoint of environmental protection, absorbent articles must have water disintegratability and/or biodegradability, but not all acrylic acid-based absorbers have satisfactory water disintegratability and biodegradability, and therefore a demand exists for new absorbers.

Absorbent cores that do not employ acrylic acid-based absorbers, such as absorbent cores employing polysaccharides, for example, are being studied to improve the problems associated with acrylic acid-based absorbers. For example, PTL 1 proposes a thickening treatment article for body fluids or excreted fluids, which comprises a polysaccharide that can increase viscosity in the presence of a polyvalent metal ion, wherein the polysaccharide is in a state in which it can dissolve or dissociate in the water of the body fluid or excreted fluid.

### [Citation List]

### [Patent Literature]

[PTL 1] Japanese Unexamined Patent Publication No. 2000-201976

A further prior art arrangement is known from EP 1129730.

### [Summary of Invention]

### [Technical Problem]

However, although the thickening treatment article for body fluids or excreted fluids disclosed in PTL 1 employs a polysaccharide capable of thickening due to the presence of a polyvalent metal ion, because it relies on a body fluid or excreted fluid as the source, the thickening effect is low with only the metal ions of the body fluid or excreted fluid, and it has the problem of insufficient retention of absorbed liquids.

A gelling agent containing a polysaccharide and polyvalent metal ion as a substitute for the acrylic acid-based absorber must therefore promote and/or inhibit gelling.

### [Solution to Problems]

As a result of much diligent research directed toward solving the aforementioned problems, the present inventors have found that the problems can be solved by an absorbent core comprising a gelling agent that contains a polysaccharide capable of thickening in the presence of a polyvalent metal ion, a substance that can supply a polyvalent metal ion, and a polyvalent metal ion scavenger, and the invention has been completed upon this finding.

Specifically, the present invention relates to the following aspects.

### [Aspect 1]

An absorbent core as recited by Claim 1.

### [Aspect 2]

The absorbent core according to aspect 1, wherein the polysaccharide capable of thickening in the presence of a polyvalent metal ion is selected from the group consisting of sodium alginate, propyleneglycol alginate, pectin, gellan gum, carrageenan, glucomannan and guar gum.

### [Aspect 3]

The absorbent core according to aspect 1 or 2, wherein the substance that can supply a polyvalent metal ion is selected from the group consisting of calcium phosphate, calcium chloride, calcium lactate, calcium gluconate, calcium acetate, aluminum sulfate, aluminum nitrate, aluminum phosphate and aluminum acetate.

### [Aspect 4]

The absorbent core according to any one of aspects 1 to 3, further comprising an organic acid. The organic acid may be selected from the group consisting of glucono-δ-lactone, adipic acid, citric acid, malic acid, tartaric acid, lactic acid and acetic acid.

### [Aspect 5]

The absorbent core according to any one of aspects 1 to 4, wherein the polyvalent metal ion scavenger is selected from the group consisting of sodium citrate, sodium polyphosphate, sodium hexametaphosphate and sodium pyrophosphate.

### [Aspect 6]

An absorbent article comprising a liquid-permeable top sheet and a liquid-impermeable back sheet, and the absorbent core according to any one of aspects 1 to 5 situated between the liquid-permeable top sheet and the liquid-impermeable back sheet.

### [Aspect 7]

The absorbent article according to aspect 6, wherein the gelling agent has a granulated structure produced by wet granulation with alcohol.

### [Aspect 8]

The absorbent article according to aspect 6 or 7, having water disintegratability.

### [Aspect 9]

The absorbent article according to any one of aspects 6 to 8, having biodegradability.

### [Advantageous Effects of Invention]

In the absorbent core of the invention that is able to promote and/or inhibit gelling, the polysaccharide capable of thickening in the presence of a polyvalent metal ion undergoes gelling of the polysaccharide after absorption of a large amount of liquid, the liquid absorption rate and retention per unit mass of polysaccharide are increased, and a smaller amount of gelling agent can be used, thus allowing the absorbent core and absorbent article to be reduced in mass and/or in thickness.

In addition, the absorbent core of the invention has low incorporation of air during dissolution and gelling, and therefore has low volume increase per unit volume upon liquid absorption, and is resistant to deformation.

Furthermore, the absorbent core of the invention accelerates gelling by addition of an organic acid, and return to the skin can be controlled in a simple manner, thus simplifying the structure of the absorbent core.

In addition, the absorbent core of the invention slows gelling by addition of a polyvalent metal ion scavenger, while increasing the liquid absorption rate per unit mass of the polysaccharide, and reduces the amount of acrylic acid-based absorber and/or pulp used, thus allowing the structure to be simplified.

### [Brief Description of Drawings]

Fig. 1 is a cross-sectional view of an absorbent core comprising a front sheet, a gelling agent-containing layer, and a rear sheet, in that order from the top.
Fig. 2 is a cross-sectional view of an absorbent core comprising a front sheet, an upper layer, a lower layer and a rear sheet in that order from the top, wherein the upper layer is a gelling agent layer.
Fig. 3 is a cross-sectional view of an absorbent core comprising a front sheet, a gelling agent-containing layer and a rear sheet in that order from the top, and being formed from two regions, a center region located at the center in the widthwise direction of the gelling agent-containing layer and/or the lengthwise direction of the absorbent core, and the peripheral regions outside of that region, where the center region is the gelling agent region.
Fig. 4 is a cross-sectional view of an absorbent core comprising a front sheet, an upper layer, a lower layer and a rear sheet in that order from the top, wherein the upper layer is the gelling agent layer and the center region of the lower layer is the gelling agent region.
Fig. 5 is a cross-sectional view of an absorbent core comprising a front sheet, an upper layer, a lower layer and a rear sheet in that order from the top, wherein the upper layer is the gelling agent layer and the peripheral regions of the lower layer are the gelling agent regions.
Fig. 6 is a cross-sectional view of an absorbent article comprising a liquid-permeable top sheet and a liquid-impermeable back sheet, and the absorbent core shown in Fig. 1 situated between the liquid-permeable top sheet and the liquid-impermeable back sheet.

### [Description of Embodiments]

The present invention will now be explained in greater detail.

### <Absorbent core>

### [Polysaccharide capable of thickening in the presence of polyvalent metal ion]

As used herein, "polysaccharide" refers to a saccharide comprising a plurality of bonded monosaccharides. Also as used herein, "polysaccharide capable of thickening in the presence of a polyvalent metal ion" refers to a polysaccharide that thickens the system in the presence of a polyvalent metal ion as described below.

Examples of polysaccharides capable of thickening in the presence of polyvalent metal ions including sodium alginate, propyleneglycol alginate, pectin, gellan gum, carrageenan, glucomannan, guar gum, locust bean gum, xanthan gum, glucose, carboxymethyl starch, mannose, galactose, arabinose, fucose, ribose, fructose and dextran. Preferred are sodium alginate, propyleneglycol alginate, pectin, gellan gum, carrageenan, glucomannan and guar gum. Sodium alginate is particularly preferred from the viewpoint of availability.

Sodium alginate is a polysaccharide produced by marine algae, and it forms the major component of sea weed dietary fiber. It dissolves in water and increases in viscosity, but can react with polyvalent metal ions to form a gel. Sodium alginate is a linear polysaccharide composed of two types of uronic acid, β-(1→4)-D-mannuronic acid (M form) and α-(1→4)-L-gluconic acid (G form), and it includes the M form consisting of M-M bonds, the G form consisting of G-G bonds, and the random form wherein M and G are randomly arranged, with the nature of the gel differing significantly depending on the proportion of the M and G forms. Because sodium alginate is naturally derived, it is biodegradable and biostable and has properties, such as liquid flow-prevention, adhesion and low abrasiveness, and is used for a wide range of purposes including food additives, adhesives, drugs, cosmetics and wound dressings

Sodium alginate is marketed in different grades with different viscosities when dissolved, and all such viscosity grades may be used in the absorbent core of the invention. From the viewpoint of easily obtaining a thickening property and/or gel strength, a high viscosity type is preferred, and it may have a viscosity of 100 mPa·s or greater and preferably 500 mPa·s or greater in a 1 mass% aqueous solution.

The amount of the polysaccharide capable of thickening in the presence of a polyvalent metal ion will differ depending on, for example, the type and molecular weight of the polysaccharide and the type and valency of the substance that can supply a polyvalent metal ion, as well as on the purpose of use of the absorbent core and the amount of liquid to be absorbed, but generally it will be 10-1,000 g/m² and preferably 50-500 g/m² as basis weight.

The form of the polysaccharide in the absorbent core of the invention may be, for example, a powder, fibrous structure, film, foam, or a complex immobilized in a base material. The polysaccharide may be used in the absorbent core in any one or more of these forms. For example, two or more forms may be used together by adjusting the timing of sol formation and/or gelling of the polysaccharide.

The powder form may be a commercially available granular form. However, when the particle sizes of the commercially available granules are small, for example, the commercially available granules may be granulated using an organic solvent or plasticizer or treated by coating or the like with a surfactant, for example, to prevent aggregation. The organic solvent used for granulation may be a lower alcohol, such as methyl alcohol, ethyl alcohol or propyl alcohol, while glycerin may be mentioned as a plasticizer that can be used for granulation.

For example, granulation can be accomplished in a simple manner by mixing the powder with the organic solvent or plasticizer for dispersion, and then drying it. Because the powder has high affinity for liquids, such as body fluids, it will tend to have undissolved portions when it is used in its original form, and it is therefore useful to perform granulation, to produce a larger particle size and to increase the surface area.

The fibrous structure may be produced by any desired method, such as, for example, spinning and drying to form it.

The film may be obtained by forming a film shape and drying it into a sheet. The film will generally be smooth and have a uniform thickness, but it may also have irregularities or a 3-dimensional structure by embossing or the like. It may also have perforations of different shapes or notches in a discontinuous pattern, such as a zigzag shape, or it may be in the form of fragmented flakes. The film may consist of a monolayer or multiple layers, and the dissolution rate or dissolution volume may vary for each layer.

The foam may be produced by any desired method, and for example, a foaming agent and/or gas and a foam stabilizer may be used to encapsulate the foam in the polysaccharide solution, and formed a film and dried it to obtain a foam body. The foaming ratio of the foamed body is not particularly restricted, and for example, foaming may be to a several-fold or several dozen-fold ratio.

The complex may be a complex of a polysaccharide and a base material, such as the polysaccharide immobilized in a base material. A binder may be used to immobilize the polysaccharide in the base material. When a binder is used, it is preferably one that does not inhibit dissolution of the polysaccharide when contacted with a liquid, such as a body fluid. As examples for the binder there may be mentioned starch, carboxymethylcellulose, polyvinyl alcohol and other water-soluble polymers, which are used as water-soluble adhesives.

The base material for the complex is not particularly restricted so long as it can hold the polysaccharide, and any desired base material may be used. The base material may be, for example, a film or sheet, or the same with perforations, notches or torn sections, or a fabric, such as a woven fabric, nonwoven fabric, knitted fabric or mesh, and preferably it is one that does not inhibit dissolution of the polysaccharide during contact with a liquid, such as a body fluid.

### [Substance that can supply polyvalent metal ion]

The "substance that can supply a polyvalent metal ion" which is included in the absorbent core of the invention is a water-soluble substance that can release a divalent or greater metal ion, such as a salt containing a divalent or greater metal ion. The polyvalent ion can crosslink the polysaccharide capable of thickening in the presence of a polyvalent metal ion, forming a three-dimensional network structure between the polyvalent metal ion and the polysaccharide capable of thickening in the presence of a polyvalent metal ion, and can gel the system. The bond between the polysaccharide capable of thickening in the presence of a polyvalent metal ion and the substance that can supply a polyvalent metal ion may be an ionic bond, for example.

As examples of divalent or greater ions there may be mentioned calcium ion and aluminum ion.

The substance that can supply a polyvalent metal ion may be, for example, a water-soluble calcium salt or a calcium salt that is water-soluble in the presence of an organic acid, such as calcium phosphates including monobasic calcium phosphate, dibasic calcium phosphate, dibasic calcium phosphate dihydrate and tribasic calcium phosphate, or calcium chloride, calcium lactate, calcium gluconate or calcium acetate, or a water-soluble aluminum salt or an aluminum salt that is water-soluble in the presence of an organic acid, such as aluminum sulfate, aluminum nitrate, aluminum phosphate or aluminum acetate

For purposes in which a fast system gelling speed is preferred there may be used a substance with high water solubility, and for purposes in which a slow system gelling speed is preferred there may be used a substance with low water solubility or with water-solubility in the presence of an organic acid.

The amount of the substance that can supply a polyvalent metal ion will differ depending on, for example, the type and valency of the substance, the type and molecular weight of the polysaccharide, and on the purpose of use of the absorbent core and the type and amount of liquid to be absorbed, but generally it will be 2-1000 g/m² and preferably 10-500 g/m² as basis weight.

The form of the substance that can supply a polyvalent metal ion, which is contained in the absorbent core of the invention, may be a powder or a complex immobilized in a base material, for example.

The powder may be commercially available granules used directly as a powder. However, when the particle sizes of the commercially available granules are small, for example, the powder may be treated as explained above under "Polysaccharide capable of thickening in the presence of polyvalent metal ion", to prevent aggregation.

Since the gelling agent in the absorbent core of the invention includes an organic acid and a polyvalent metal ion scavenger, described below, it is possible to promote and/or inhibit formation of the three-dimensional network structure, as desired. However, by using a method of coating the substance that can supply a polyvalent metal ion with a surfactant, gelatin, wafer sheet or the like, or covering it with microcapsules or the like, it is possible to control dissolution of the substance that can supply a polyvalent metal ion, and thus control the timing of formation of the three-dimensional network structure. This method can be used for the absorbent core of the invention, as a supplementary gel-inhibiting method.

The complex may be a complex of the substance that can supply a polyvalent metal ion with a base material, such as one wherein the substance that can supply a polyvalent metal ion is immobilized in a base material. A binder may be used to immobilize the substance that can supply a polyvalent metal ion in the base material. When a binder is used, it is preferably one that does not inhibit dissolution of the substance that can supply a polyvalent metal ion when it is contacted with a liquid, such as a body fluid. As examples for the binder there may be mentioned starch, carboxymethylcellulose, polyvinyl alcohol and other water-soluble polymers, which are used as water-soluble adhesives.

The base material is not particularly restricted so long as it can hold the substance that can supply a polyvalent metal ion, and any desired base material may be used. As examples for the base material there may be mentioned fabrics, such as woven fabrics, nonwoven fabrics, films and sheets, and those mentioned above under "Polysaccharide capable of thickening in the presence of polyvalent metal ion" may be used.

### [Organic acid]

As used herein, "organic acid" refers to an organic compound that can lower the pH of the system. As examples of organic acids to be used in the absorbent core of the invention there may be mentioned glucono-δ-lactone, adipic acid, citric acid, malic acid, tartaric acid, lactic acid and acetic acid. The organic acid may be used alone or in combinations of two or more.

The following is the mechanism by which the organic acid promotes formation of a three-dimensional network structure between the polysaccharide capable of thickening in the presence of a polyvalent metal ion and the polyvalent metal ion.
(1) Each component of the gelling agent begins to dissolve in the liquid that has permeated the absorbent core,
(2) the polysaccharide capable of thickening in the presence of a polyvalent metal ion dissolves, thus increasing the viscosity of the liquid and inhibiting the flow property,
(3) the organic acid dissolves and lowers the pH of the liquid, and especially when glucono-δ-lactone is used as the organic acid, equilibrium reaction takes place with gluconic acid, thus gradually lowering the pH of the solution, and
(4) the lowered pH promotes release of the polyvalent metal ion from the substance that can supply a polyvalent metal ion, and formation of a three-dimensional network structure between the released polyvalent metal ion and the polysaccharide capable of thickening in the presence of a polyvalent metal ion.

The amount of organic acid is not particularly restricted and will differ depending on, for example, the types and amounts of the polysaccharide capable of thickening in the presence of a polyvalent metal ion, the substance that can supply a polyvalent metal ion and the polyvalent metal ion scavenger, but it will generally be an amount that can lower the pH of the system, adjust the dissolution rate of the substance that can supply a polyvalent metal ion, and promote formation of a three-dimensional network structure, and it may be determined as appropriate.

The form of the organic acid, which is contained in the absorbent core of the invention, may be a powder or a complex immobilized in a base material, for example.

The powder may be commercially available granules used directly as a powder. However, when the particle sizes of the commercially available granules are small, for example, the powder may be treated as explained above under "Polysaccharide capable of thickening in the presence of polyvalent metal ion", to prevent aggregation.

The complex may be a complex of an organic acid and a base material, such as the organic acid polysaccharide immobilized in a base material. A binder may be used to immobilize the organic acid in the base material. When a binder is used, it is preferably one that does not inhibit dissolution of the organic acid when contacted with a liquid, such as a body fluid. As examples for the binder there may be mentioned starch, carboxymethylcellulose, polyvinyl alcohol and other water-soluble polymers, which are used as water-soluble adhesives.

The base material is not particularly restricted so long as it can hold the organic acid, and any desired base material may be used. As examples for the base material there may be mentioned fabrics, such as woven fabrics, nonwoven fabrics, films and sheets, and those mentioned above under "Polysaccharide capable of thickening in the presence of polyvalent metal ion" may be used.

### [Polyvalent metal ion scavenger]

As used herein, "polyvalent metal ion scavenger" refers to, for example, a compound that scavenges the substance that can supply a polyvalent metal ion, or a polyvalent metal ion from a liquid, such as a body fluid, or for example, a compound that forms a complex with the polyvalent metal ion. The polyvalent metal ion scavenger used in the absorbent core of the invention may be, for example, a carboxylic acid salt, such as sodium citrate or a phosphoric acid salt, such as sodium polyphosphate, sodium hexametaphosphate or sodium pyrophosphate.

The following is the mechanism by which the polyvalent metal ion scavenger inhibits formation of a three-dimensional network structure between the polysaccharide capable of thickening in the presence of a polyvalent metal ion and the substance that can supply a polyvalent metal ion.
(1) Each component of the gelling agent begins to dissolve in the liquid that has permeated the absorbent core,
(2) the polysaccharide capable of thickening in the presence of a polyvalent metal ion dissolves, thus increasing the viscosity of the liquid and inhibiting the flow property (thickening),
(3) the dissolved polyvalent metal ion scavenger sequesters the polyvalent metal ion released from the substance that can supply a polyvalent metal ion (for example, forming a complex with the polyvalent metal ion), and
(4) sequestering of the polyvalent metal ion prevents the polyvalent metal ion concentration in the solution from rising, and inhibits formation of a three-dimensional network structure.

The amount of the polyvalent metal ion scavenger is not particularly restricted, and it will differ depending on the type and amount of the polysaccharide capable of thickening in the presence of a polyvalent metal ion, the substance that can supply a polyvalent metal ion and the organic acid that are used.

The form of the polyvalent metal ion scavenger, which is contained in the absorbent core of the invention, may be a powder or a complex immobilized in a base material, for example.

The powder may be commercially available granules used directly as a powder. However, when the particle sizes of the commercially available granules are small, for example, the powder may be treated as explained above under "Polysaccharide capable of thickening in the presence of polyvalent metal ion", to prevent aggregation.

The complex may be a complex of the polyvalent metal ion scavenger with a base material, such as one wherein the polyvalent metal ion scavenger is immobilized in a base material. A binder may be used to immobilize the polyvalent metal ion scavenger in the base material. When a binder is used, it is preferably one that does not inhibit dissolution of the polyvalent metal ion scavenger when it is contacted with a liquid, such as a body fluid. As examples for the binder there may be mentioned starch, carboxymethylcellulose, polyvinyl alcohol and other water-soluble polymers, which are used as water-soluble adhesives.

The base material is not particularly restricted so long as it can hold the polyvalent metal ion scavenger, and any desired base material may be used. As examples for the base material there may be mentioned fabrics, such as woven fabrics, nonwoven fabrics, films and sheets, and those mentioned above under "Polysaccharide capable of thickening in the presence of polyvalent metal ion" may be used.

### [Gelling agent]

As used herein, a "gelling agent" contains a polysaccharide capable of thickening in the presence of a polyvalent metal ion, a substance that can supply a polyvalent metal ion, an organic acid and/or a polyvalent metal ion scavenger.

Each component of the gelling agent in the absorbent core of the invention, i.e. the polysaccharide capable of thickening in the presence of a polyvalent metal ion, the substance that can supply a polyvalent metal ion, and an organic acid and/or a polyvalent metal ion scavenger, may be separately introduced into the absorbent core. When the components are separately introduced, each component may be introduced in their respective forms described above.

The polysaccharide capable of thickening in the presence of a polyvalent metal ion, the substance that can supply a polyvalent metal ion, and an organic acid and/or a polyvalent metal ion scavenger, may also be mixed to prepare a gelling agent, and then introduced into the absorbent core. The form of the gelling agent that is introduced, when a gelling agent is prepared beforehand, may be, for example, a powder, fibrous structure, film, foam, or a complex immobilized in a base material.

The powder, fibrous structure, film, foam or complex immobilized in a base material may be in the same form as explained above under "Polysaccharide capable of thickening in the presence of polyvalent metal ion".

### [Absorbent core]

As used herein, "absorbent core" refers to products that serve mainly for absorption of liquids and which are used for purposes that include sanitary products, such as sanitary napkins and panty liners, sanitary materials, such as disposable diapers, urine leakage-preventing sheets, urine-absorbing pads for incontinent patients, body fluid/blood-absorbing medical goods, wound-dressing materials, cosmetic pack materials, animal excrement-treating materials, agricultural and gardening products, freshness-keeping materials for foods, moisture condensation-proof materials and products to be used in locations that require moisture absorption and/or moisture retention.

The absorbent core of the invention may comprise a gelling agent and a base material in the interior, a front sheet on the front side (absorbing side) and a rear sheet on the rear side. When the absorbent core of the invention comprises a multilayer structure or multiple regions, the absorbent core of the invention may comprise an interlayer sheet between each layer and between each region.

The base material in the absorbent core may be, for example, a fiber or nonwoven fabric. The base material preferably has water disintegratability and/or biodegradability.

As examples of fiber materials in the absorbent core of the invention there may be mentioned those formed of synthetic fibers, such as polyester fiber, polyacrylonitrile fiber, polyvinyl chloride fiber, polyethylene fiber, polypropylene fiber, polystyrene fiber, polyethylene terephthalate fiber, polyethylene/polypropylene fiber, polyethylene/polyethylene terephthalate composite fiber and polyvinyl alcohol fiber, semisynthetic fibers, such as cellulose-based fiber and protein-based fiber, natural fibers, regenerated fibers, such as cellulose-based fiber including pulp fiber, and rayon fiber, acetate fiber and the like. The fiber may consist of staple fibers or filaments, and may have any desired diameter. The fiber may also be, for example, core-sheath composite fiber, parallel composite fiber, heterogeneous cross-section hollow fiber, microporous fiber, conjugated composite fiber or the like.

The nonwoven fabric used in the absorbent core of the invention, or in the front sheet or interlayer sheet, may be a known nonwoven fabric, such as, for example, a thermal bonded nonwoven fabric, a melt-blown nonwoven fabric, a spunbond/melt-blown/spunbond nonwoven fabric, a spunlace nonwoven fabric, an airlaid nonwoven fabric, a through-air nonwoven fabric (TA) or a point bond nonwoven fabric (PB). A water disintegratable tissue may also be used.

The method of forming the nonwoven fabric may be, for example, a dry or wet method, such as a through-air, point bond or spunlace method.

The nonwoven fabric may also have an elastic property. As elastic nonwoven fabrics there may be mentioned nonwoven fabrics produced by meltblown or spunbond methods. The elastic nonwoven fabric may be produced from elastic fibers obtained by melting and spinning a thermoplastic elastomer resin.

The rear sheet of the absorbent core of the invention may be one produced from a polyester, polyacrylonitrile, polyvinyl chloride, polyethylene, polypropylene, polystyrene, polyethylene terephthalate or polyvinyl alcohol, for example. There may also be mentioned air-permeable films, non-air-permeable films, porous films and the like made from high-density polyethylene/low-density polyethylene.

The absorbent core of the invention may further include various additives including thickeners, plasticizers, aromas, deodorants, inorganic powders, pigments, dyes, antimicrobial materials, pressure-sensitive adhesives and the like, which are commonly used in the art. As examples of thickeners there may be mentioned polyvinyl alcohol and polyacrylic acid, and as examples of plasticizers there may be mentioned glycerin, sorbitol, lactitol, maltitol, erythritol and pentaerythritol. Trehalose may also be used to prevent cracks of the film or sheet during drying. Such additives can impart various functions to the absorbent core. As examples of inorganic powders there may be mentioned substances that are inert with respect to the liquid, such as silicon dioxide, zeolite, kaolin and clay.

The additives mentioned above may be added to the absorbent core of the invention by methods commonly used in the art.

The structure of the absorbent core of the invention is not particularly restricted so long as it is a structure including the gelling agent, and any structure may be employed.

The construction of the absorbent core of the invention will now be explained with reference to the accompanying drawings. It should be noted that the aspects depicted in the drawings are examples, and do not restrict the invention in any way.

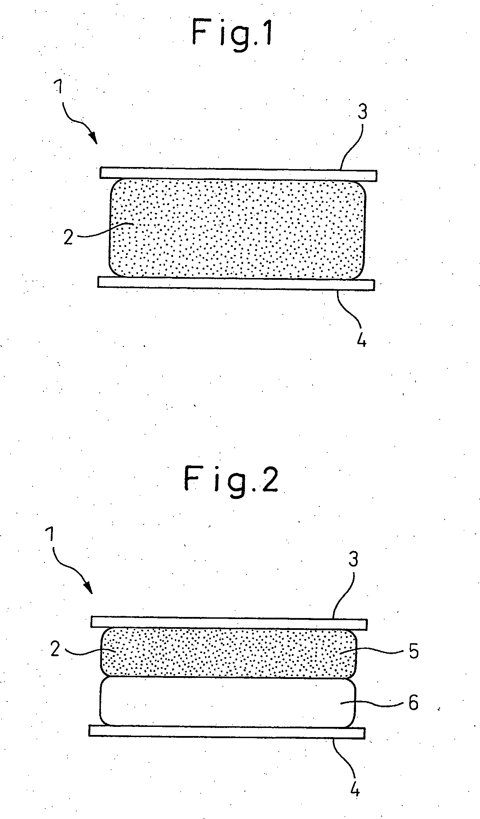

Figs. 1 to 5 are all cross-sectional views of absorbent core 1, in which the top side is the front side, i.e. the side that contacts with a human.

Fig. 1 is a cross-sectional view of absorbent core 1 comprising front sheet 3, gelling agent layer 2, and rear sheet 4, in that order from the top.

Fig. 2 is a cross-sectional view of absorbent core 1 comprising front sheet 3, upper layer 5, lower layer 6 and rear sheet 4 in that order from the top, wherein upper layer 5 is gelling agent layer 2. Lower layer 6 is not particularly restricted and may be another absorbing material, such as a pulp layer, or if it is necessary to absorb a large amount of liquid, it may be an acrylic acid-based absorber layer. By providing the construction shown in Fig. 2 it is possible to effectively prevent return of liquid on the front side. The layers in absorbent core 1 may be 3 or more layers, and pulp and acrylic acid-based absorber layers may be situated in the middle layer and lower layer.

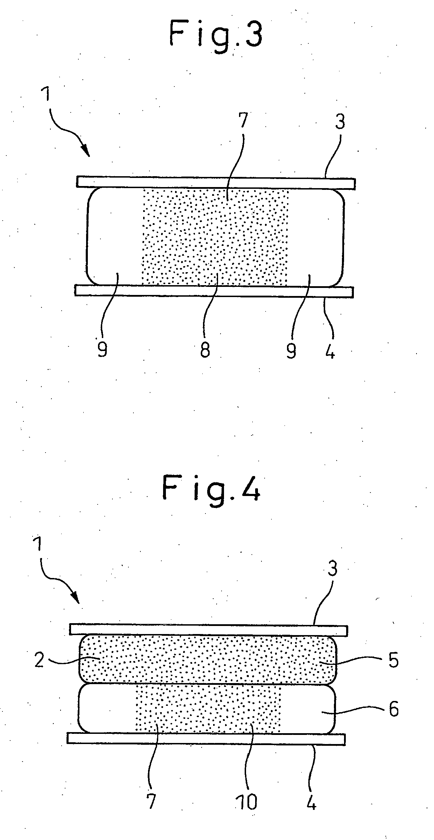

Fig. 3 is a cross-sectional view of absorbent core 1 comprising front sheet 3, a gelling agent-containing layer and rear sheet 4 in that order from the top, and being formed from one or two regions, center region 8 located at the center in the widthwise direction of the gelling agent-containing layer and/or the lengthwise direction of the absorbent core, and the peripheral regions 9 outside of that region, where center region 8 is gelling agent region 7. By providing the construction shown in Fig. 3 it is possible to effectively prevent return of liquid near urinating locations. The regions in absorbent core 1 may be 3 or more regions, and pulp and acrylic acid-based absorber regions may be located in the regions other than the gelling agent region.

Fig. 4 is a cross-sectional view of absorbent core 1 comprising front sheet 3, upper layer 5, lower layer 6 and rear sheet 4 in that order from the top, wherein upper layer 5 is gelling agent layer 2 and center region of lower layer 10 is gelling agent region 7. By providing this construction, it is possible to effectively prevent return of liquid on the front side and especially to effectively prevent return of liquid near urinating locations.

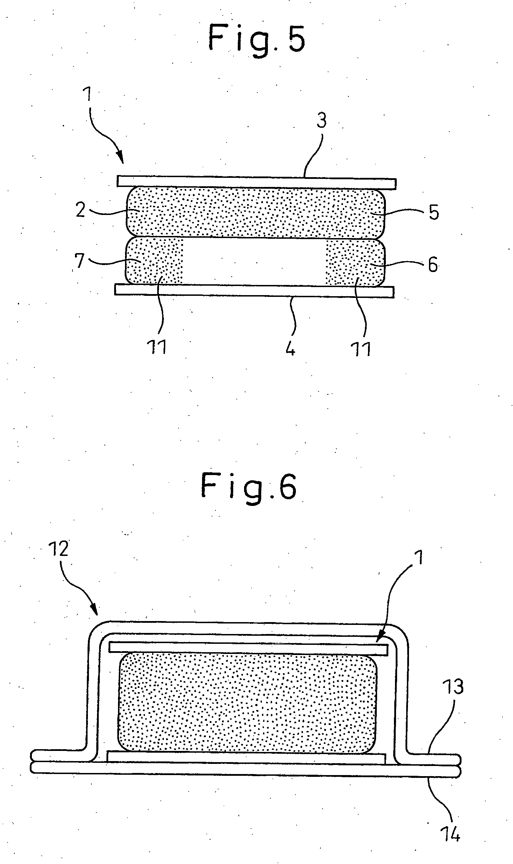

Fig. 5 is a cross-sectional view of absorbent core 1 comprising front sheet 3, upper layer 5, lower layer 6 and rear sheet 4 in that order from the top, wherein upper layer 5 is gelling agent layer 2 and peripheral regions of lower layer 11 are the gelling agent regions 7. By providing this construction, it is possible to effectively prevent return of liquid on the front side and especially to effectively prevent return of liquid near perimeter sections.

Also, the absorbent core of the invention may be composed of 2 layers or 3 or more layers, with the amount of organic acid and/or polyvalent metal ion scavenger varying between each layer. For example, the upper layer may have a higher organic acid concentration than the lower layer, to accelerate gelling and effectively prevent return of liquid. Also, the polyvalent metal ion scavenger concentration of the upper layer may be higher than the lower layer to slow gelling of the upper layer, promote permeation of more liquid in the lower layer, and increase the overall amount of absorption.

Also, the absorbent core of the invention may be divided into 2, 3 or more regions, so as to create a center region and peripheral regions, for example, with the amount of organic acid and/or polyvalent metal ion scavenger varying between each region. For example, a greater amount of organic acid may be added to the center region than the peripheral regions, to effectively prevent return of liquid. Also, a greater amount of polyvalent metal ion scavenger may be added to the center region than the peripheral regions, to slow gelling in the center region, promote permeation of more liquid in the peripheral region, and increase the overall amount of absorption.

Also, the absorbent core of the invention may be composed of 2, 3 or more layers and at least one layer may be divided into 2, 3 or more regions, with the amount of organic acid and/or polyvalent metal ion scavenger varying between each layer and/or between each region, as explained above.

Since the gelling agent of the absorbent core of the invention has water disintegratability, the front sheet and rear sheet may be selected from among water disintegratable materials to impart water disintegratability to the absorbent core.

Furthermore, since the gelling agent of the absorbent core of the invention is biodegradable, the front sheet and rear sheet may be selected from among biodegradable materials to impart a biodegradable property to the absorbent core.

### <Absorbent article>

As used herein, "absorbent article" refers to sanitary products, such as sanitary napkins and panty liners, sanitary materials, such as disposable diapers, urine leakage-preventing sheets, urine-absorbing pads for incontinent patients, body fluid/blood-absorbing medical goods, wound-dressing materials, cosmetic pack materials, animal excrement-treating materials, agricultural and gardening products, freshness-keeping materials for foods, moisture condensation-proof materials and articles to be used in locations that require moisture absorption and/or moisture retention.

As used herein, "liquid-permeable top sheet" refers to a sheet situated on the top side (absorbing side), which allows permeation of liquids and particularly body fluids. The liquid-permeable top sheet used in the absorbent article of the invention is not particularly restricted as long as it is a sheet which is permeated by liquids, and as examples there may be mentioned hydrophilic fiber nonwoven fabrics, hydrophobic fiber nonwoven fabrics with multiple openings, and plastic films with openings.

As used herein, "liquid-impermeable back sheet" refers to a sheet situated on the back side (clothing side), which does not allow permeation of liquids and particularly body fluids, under ordinary conditions of use. The liquid-impermeable back sheet used in the absorbent article of the invention is not particularly restricted so long as it is a sheet that is not permeated by liquids, and it may be any sheet used in the art, such as a film or nonwoven fabric.

The structure of the absorbent article of the invention is not particularly restricted as long as the absorbent core can effectively absorb liquids, and particularly body fluids or liquid excreted fluids, and it may have any desired form used in the art, such as the following aspects, for example.
(a) An absorbent article comprising a liquid-permeable top sheet and a liquid-impermeable back sheet, and an absorbent core situated between the liquid-permeable top sheet and the liquid-impermeable back sheet.
(b) An absorbent article comprising a liquid-permeable top sheet and a liquid-impermeable back sheet, and an absorbent core situated between the liquid-permeable top sheet and the liquid-impermeable back sheet, wherein a material that aids diffusion and/or absorption is further situated between the front sheet and the absorbent core and/or between the absorbent core and the rear sheet.
(c) An absorbent article according to (a) or (b) above, wherein either or both the liquid-permeable top sheet and liquid-impermeable back sheet have water disintegratability.
(d) An absorbent article according to any one of (a) to (c) above, wherein either or both the liquid-permeable top sheet and liquid-impermeable back sheet are biodegradable.

Pulp and high water absorption resins may be mentioned as examples of materials that aid diffusion and/or absorption.

The construction of the absorbent article of the invention will now be explained with reference to the accompanying drawings. It should be noted that the aspects depicted in the drawings are examples, and do not restrict the invention in any way.

Fig. 6 is a cross-sectional view of absorbent article 12 comprising liquid-permeable top sheet 13 and liquid-impermeable back sheet 14, and the absorbent core 1 shown in Fig. 1 situated between liquid-permeable top sheet 13 and liquid-impermeable back sheet 14.

### [Examples]

The present invention will now be explained in detail through the following examples, with the understanding that these examples are not limitative on the invention.

### [Production Example 1]

Sodium alginate (B-S, by Kimica Corp.), dibasic calcium phosphate dihydrate (Wako Pure Chemical Industries, Ltd.) and glucono-δ-lactone (Wako Pure Chemical Industries, Ltd.) were mixed to uniformity in a mass ratio of 1:1:1, to prepare a gelling agent. The gelling agent was immobilized over the entire surface of an air-through nonwoven fabric (PE/PP core-sheath composite fiber, basis weight: 40 g/m²) by spraying water with a sprayer basis weight: 150 g/m²), to produce a gelling agent sheet. The gelling agent sheet was placed on a rear sheet (polyethylene film) and then a front sheet (through-air nonwoven fabric, PE/PP core-sheath composite fiber, basis weight: 25 g/m²) was situated thereover, to produce an absorbent core.

### [Gelling speed control examples: Examples 1 to 9]

A polysaccharide capable of thickening in the presence of a polyvalent metal ion, a substance that can supply a polyvalent metal ion, and an organic acid and/or a polyvalent metal ion scavenger were mixed according to the compositions listed in Table 1. Upon visually confirming uniformity of the mixture, 20 g of artificial urine (prepared by dissolving 2% urea, 0.8% sodium chloride, 0.08% magnesium sulfate heptahydrate and 0.03% calcium chloride dihydrate in ion-exchanged water) was added to the mixture while stirring, the state of the contents was observed during a maximum of 30 minutes while continuing to stir, and the flow control time (sol-forming time) and gelling time were measured.

For Example 5, sodium alginate, dibasic calcium phosphate, glucono-δ-lactone and ethanol were mixed and dried, for wet granulation of the gelling agent. The granulated gelling agent had a larger (visually apparent) particle size than the gelling agent produced in Example 1, and more excellent handling properties.

The results are shown in Table 1. In Table 1, "Flow control time" means the time from addition of the artificial urine until thickening of the gelling agent.

### [Gelling speed control examples - Comparative Example 1 and Comparative Example 2]

The flow control time and gelling time were measured in the same manner as Example 1, except that a polysaccharide capable of thickening in the presence of a polyvalent metal ion and a substance that can supply a polyvalent metal ion were used according to Table 1.

[Table 1]

**Table 1. Gelling agent compositions and results**

| | Ex. 1 | Ex. 2 | Ex. 3 | Ex. 4 | Ex. 5 | Ex. 6 | Ex. 7 | Ex. 8 | Ex. 9 | Comp. Ex. 1 | Comp. Ex. 2 |
|---|---|---|---|---|---|---|---|---|---|---|---|
| Polysaccharide capable of thickening in presence of polyvalent metal ion | 0.5 | 0.5 | 0.5 | 0.5 | 0.5 | 0.5 | 0.5 | 0.5 | 0.5 | 0.5 | 0.5 |
| Sodium alginate (G) | | | | | | | | | | | |
| Substance that can supply polyvalent metal ion | 0.5 | 0.5 | 0.5 | 0.5 | 0.5 | 0.5 | - | - | - | 0.5 | - |
| Dibasic calcium phosphate dihydrate (g) | | | | | | | | | | | |
| Calcium lactate (g) | - | - | - | - | - | - | 0.5 | 0.5 | 0.5 | - | 0.5 |
| Organic acid | 0.5 | 1.0 | 2.0 | 3.0 | 0.5 | 0.5 | - | - | - | - | - |
| Glucono-δ-lactone (g) | | | | | | | | | | | |
| Polyvalent metal ion scavenger | - | - | - | - | - | 0.05 | 0.05 | 0.1 | 0.5 | - | - |
| Sodium pyrophosphate (g) | | | | | | | | | | | |
| Ethanol (g) | - | - | - | - | 1.0 | - | - | - | - | - | - |
| Flow control time (min) | 1 | 1 | 1 | 1 | 1 | 20 | | | 1 | | 1 |
| Gelling time (min) | 10 | 5 | 3 | 3 | 5 | - | *1 | *1 | - | - | *1 |

| | | | | | | | | | | | |
|---|---|---|---|---|---|---|---|---|---|---|---|
| *1: Sodium alginate gelled upon incorporating a portion of the artificial urine, and a portion of the artificial urine remained without gelling, making it impossible to evaluate gelling time. | | | | | | | | | | | |

From Examples 1-3 and Comparative Example 1 it is seen that addition of an organic acid resulted in no change in flow control time but an accelerated gelling time. Thus, addition of an organic acid can promote and/or inhibit the gelling time. Based on Example 1 and Example 5 it is seen that using ethanol for granulation of the gelling agent reduces the gelling time by about half, from 10 minutes to 5 minutes.

From Example 1 and Example 6 it is seen that addition of an ion scavenger can slow the flow control time.

A comparison of Examples 7-9 and Comparative Example 2 shows that when highly water-soluble calcium lactate is used as the substance that can supply a polyvalent metal ion, addition of an ion scavenger can inhibit gelling.

### [Industrial Applicability]

The absorbent core of the invention can be used in sanitary products, such as sanitary napkins and panty liners, sanitary materials, such as disposable diapers, urine leakage-preventing sheets, urine-absorbing pads for incontinent patients, body fluid/blood-absorbing medical goods, wound-dressing materials, cosmetic pack materials, animal excrement-treating materials, agricultural and gardening products, freshness-keeping materials for foods, moisture condensation-proof materials and articles to be used in locations that require moisture absorption and/or moisture retention.

### [Explanation of Symbols]

- 1: Absorbent core
- 2: Gelling agent layer
- 3: Front sheet
- 4: Rear sheet
- 5: Upper layer
- 6: Lower layer
- 7: Gelling agent region
- 8: Center region
- 9: Peripheral region
- 10: Center region of lower layer
- 11: Peripheral region of lower layer
- 12: Absorbent article
- 13: Liquid-permeable top sheet
- 14: Liquid-impermeable back sheet

## Claims

1. An absorbent core (1) comprising a gelling agent **characterized in that** the gelling agent contains a polysaccharide capable of thickening in the presence of a polyvalent metal ion, a substance that can supply a polyvalent metal ion, and a polyvalent metal ion scavenger.

2. The absorbent core according to claim 1, wherein the polysaccharide capable of thickening in the presence of a polyvalent metal ion is selected from the group consisting of sodium alginate, propyleneglycol alginate, pectin, gellan gum, carrageenan, glucomannan and guar gum.

3. The absorbent core according to claim 1 or 2, wherein the substance that can supply a polyvalent metal ion is selected from the group consisting of calcium phosphate, calcium chloride, calcium lactate, calcium gluconate, calcium acetate, aluminum sulfate, aluminum nitrate, aluminum phosphate and aluminum acetate.

4. The absorbent core according to any preceding claim further comprising an organic acid.

5. The absorbent core according to Claim 4, wherein the organic acid is selected from the group consisting of glucono-δ-lactone, adipic acid, citric acid, malic acid, tartaric acid, lactic acid and acetic acid.

6. The absorbent core according to any one of claims 1 to 5, wherein the polyvalent metal ion scavenger is selected from the group consisting of sodium citrate, sodium polyphosphate, sodium hexametaphosphate and sodium pyrophosphate.

7. An absorbent article comprising a liquid-permeable top sheet (13) and a liquid-impermeable back sheet (14), and the absorbent core (1) according to any one of claims 1 to 6 situated between the liquid-permeable top sheet and the liquid-impermeable back sheet.

8. The absorbent article according to claim 7, wherein the gelling agent has a granulated structure produced by wet granulation with alcohol.

9. The absorbent article according to claim 7 or 8, having water disintegratability.

10. The absorbent article according to any one of claims 7 to 9, having biodegradability.

## Patentansprüche

1. Saugfähinges Innenteil (1), das ein Geliermittel umfasst, **dadurch gekennzeichnet, dass** das Geliermittel ein Polysaccharid enthält, das bei Vorhandensein eines Mehrfachmetallions zum Verdicken in der Lage ist, eine Substanz, die ein Mehrfachmetallion und eine Mehrfachmetallion-Abbauzelle bereitstellen kann.

2. Saugfähiges Innenteil nach Anspruch 1, wobei das Polysaccharid, das bei Vorhandensein eines Mehrfachmetallions zum Verdicken in der Lage ist, von der Gruppe gewählt wird, die Natrumalginat, Propylenglykolalginat, Pektin, Gellangummi, Carragenan, Glukomanna und Guaran enthält.

3. Saugfähiges Innenteil nach Anspruch 1 oder 2, wobei die Substanz, die ein Polyvalentmetallion bereitstellen kann, von der Gruppe gewählt wird, die Kalziumphosphat, Kalziumchlorid, Kalziumlaktak, Kalziumgluconat, Kalziumacetat, Aluminiumsulfat, Aluminiumnitrat, Aluminiumphosphat, und Aluminiumacetat enthält.

4. Saugfähiges Innenteil nach einem vorangehenden Anspruch, der ferner eine organische Säure umfasst.

5. Saugfähiges Innenteil nach Anspruch 4, wobei die organische Säure von einer Gruppe gewählt wird, die Glucono-delta-Lacton, Adipinsäure, Zitronensäure, Apfelsäure, Weinsäure, Milchsäure und Ethansäure enthält.

6. Saugfähiges Innenteil nach einem der Ansprüche 1 bis 5, wobei die Mehrfachmetallions-Abbauzelle von der Gruppe gewählt wird, die Natriumcitrat, Natriumphosphat, Natriumhexametaphosphat und Natriumpyrophosphat enthält.

7. Saugfähiger Artikel, der eine flüssigkeitsdurchlässige oberste Schicht (13) und eine flüssigkeitsundurchlässige hintere Schicht (14) umfasst, und das saugfähige Innenteil (1) nach einem der Ansprüche 1 bis 6 zwischen der flüssigkeitsdurchlässigen obersten Schicht und der flüssigkeitsundurchlässigen hinteren Schicht angebracht ist.

8. Saugfähiger Artikel nach Anspruch 7, wobei das Geliermittel eine granulierte Struktur hat, die von nasser Granulation mit Alkohol erzeugt wird.

9. Saugfähiger Artikel nach Anspruch 7 oder 8, der eine Wasserauflösbarkeit hat.

10. Saugfähiger Artikel nach einem der Ansprüche 7 bis 9, der eine biologische Abbaubarkeit hat.

## Revendications

1. Partie centrale absorbante (1) comportant un agent gélifiant, **caractérisée en ce que** l'agent gélifiant contient un polysaccharide en mesure d'épaissir en présence d'un ion de métal polyvalent, une substance qui peut fournir un ion de métal polyvalent, et un capteur d'ion de métal polyvalent.

2. Partie centrale absorbante selon la revendication 1, dans laquelle le polysaccharide en mesure d'épaissir en présence d'un ion de métal polyvalent est sélectionné dans le groupe constitué par de l'alginate de sodium, de l'alginate de glycol propylénique, de la pectine, de la gomme gellane, du carraghénane, du glucomannane et de la gomme de guar.

3. Partie centrale absorbante selon la revendication 1 ou la revendication 2, dans laquelle la substance qui peut fournir un ion de métal polyvalent est sélectionnée dans le groupe constitué par du phosphate de calcium, du chlorure de calcium, du lactate de calcium, du gluconate de calcium, de l'acétate de calcium, du sulfate d'aluminium, du nitrate d'aluminium, du phosphate d'aluminium et de l'acétate d'aluminium.

4. Partie centrale absorbante selon l'une quelconque des revendications précédentes, comportant par ailleurs un acide organique.

5. Partie centrale absorbante selon la revendication 4, dans laquelle l'acide organique est sélectionné dans le groupe constitué par de la glucono-δ-lactone, de l'acide adipique, de l'acide citrique, de l'acide malique, de l'acide tartrique, de l'acide lactique et de l'acide acétique.

6. Partie centrale absorbante selon l'une quelconque des revendications 1 à 5, dans laquelle le capteur d'ion de métal polyvalent est sélectionné dans le groupe constitué par du citrate de sodium, du polyphosphate de sodium, de l'héxamétaphosphate de sodium et du pyrophosphate de sodium.

7. Article absorbant comportant une feuille de dessus perméable aux liquides (13) et une feuille de support imperméable aux liquides (14), et la partie centrale absorbante (1) selon l'une quelconque des revendications 1 à 6 située entre la feuille de dessus perméable aux liquides et la feuille de support imperméable aux liquides.

8. Article absorbant selon la revendication 7, dans lequel l'agent gélifiant a une structure granulée produite par une opération de granulation par voie humide avec de l'alcool.

9. Article absorbant selon la revendication 7 ou la revendication 8, ayant une aptitude à la désintégration dans l'eau.

10. Article absorbant selon l'une quelconque des revendications 7 à 9, ayant une aptitude à la biodégradation.
